# FASCICULE DE BREVET EUROPEEN

(11) **EP 0 541 737 B1**
(45) Date de publication et mention de la délivrance du brevet: **09.04.1997**
(21) Numéro de dépôt: 92908925.8
(22) Date de dépôt: 01.05.1992
(51) Int. Cl.: C07C 235/38, A61K 7/42

(54) **LES N-PHENYL-CINNAMAMIDES, PROTECTEURS CONTRE LES EFFETS NOCIFS DE LA LUMIERE ULTRA-VIOLETTE**
N-PHENYL-CINNAMAMIDE, DIE EINEN SCHUTZ VERLEIHEN GEGEN SCHÄDIGENDE EFFEKTE ULTRAVIOLETTEN LICHTES
N-PHENYL-CINNAMAMIDES PROVIDING PROTECTION FROM THE HARMFUL EFFECTS OF ULTRAVIOLET LIGHT

(30) Priorité: 02.05.1991 CH 1305/91
(43) Date de publication de la demande: 19.05.1993
(73) Titulaire: BAUDET, Pierre, CH-1234 Vessy (CH)
(72) Inventeur: BAUDET, Pierre, CH-1234 Vessy (CH)
(86) Numéro de dépôt international: CH9200088
(87) Numéro de publication internationale: WO9219223

(56) Documents cités:
- EP-A- 0 095 097
- EP-A- 0 124 791
- EP-A- 0 235 064
- US-A- 4 337 270
- US-A- 4 883 653
- CHEMICAL ABSTRACTS, vol. 83, no. 8, 25 août 1975, Columbus, Ohio, US; abstract no. 65337y, page 436; & JP-A-7 469 874

## Description

La lumière solaire,au niveau de l'écorce terrestre,comprend une partie de lumière ultra-violette dont le spectre s'étend de 280 à 400 nm.Les radiations de longueur d'onde plus courte sont retenues par la couche d'ozone circumterreste,dans la haute atmosphère.
La lumière ultra-violette des courtes longueurs d'ondes à 280 nm est appelée UV-C,de 280 à 320 nm UV-B et de 320 à 400 nm UV-A. L'irradiation d'un organisme vivant par les UV-C est léthale; l'irradiation par les UV-B est toxique,provoquant de l'érythème et des lésions du stratum corneum:l'irradiation par les UV-A provoque le brunissement de la peau en induisant la mélanogenèse dans les mélanocytes.La mélanine formée à partir de la dopamine provenant de la tyrosine est transférée dans le kératocytes.Le brunissement rend l'épiderme moins sensible à l'effet toxique des UV-A qui pénétrent dans le derme.Il n'empêche cependant pas les UV-A de provoquer le vieillissement de la peau et l'induction de carcinomes cutanés.
La quantité de UV-A parvenant sur la surface terrestre est env.1000 fois plus importante que la quantité de UV-B.
L'organisme humain,en particulier les téguments(peau et cheveux) doit être protégé contre les effets néfastes des radiations UV-A et UV-B de la lumière solaire et celà d'autant plus que la populaticn est de race blanche et qu'elle vit sur des continents à forte exposition solaire ou qu'elle s'expose momentanément à de fortes irradiations solaires.
Pour réaliser cette protection on a créé des produits cosmétiques, appelés filtres ou écrans solaires,contenant des substances inorganiques ou organiques qui réfléchissent ou absorbent respectivement la lumière UV-A et UV-B.Les substances minérales sont le talc,l'oxyde de zinc et l'oxyde de titane,on les appelle des écrans solaires. On leurs préfére pour l'esthétique des solutions translucides ou laiteuses de molécules organiques,les filtres solaires,en solution dans des crèmes,des laits,des lotions,des huiles,des gels,à raison d'une concentration de 0,5 à 10 %.Les molécules organiques sont choisies en fonction de leur pouvoir dábsorption de la lumière ultra-violette UV-A et UV-B et de leur inocuité.
Dans le tableau I nous présentons les principaux filtres solaires commercialisés,en indiquant leur longueur d'onde maximum de leur spectre d'absorption UV A et UV-B (λ max.) et l'intensité de leur pouvoir d'absorption des UV-A et UV-B,exprimé en coefficient d'extinction moléculaire ( ε max.),leur appartenance à la classe des UV.

**Tableau I**

| filtres | λ max. (nm) | ε max | UV |
|---|---|---|---|
| 1) acide para-amino-benzoique | 283 | 15300 | B |
| 2) para-diméthylamino-benzoate d'octyle | 311 | 27300 | B |
| 3) 4-méthoxy-2-hydroxy-benzophénone | 288 | 14000 | B |
| | 325 | 9400 | A |
| 4) 4-méthoxy-2-hydoxy-3-sulfo-benzophénone | 286 | 13400 | B |
| | 325 | 8400 | A |
| 5) 2,2'-dihydroxy-4-méthoxy-benzophénone | 284 | 13270 | B |
| | 327 | 10440 | A |
| 6) 4-méthoxy-cinnamate de 2-éthyl-héxyle | 311 | 23300 | B |
| 7) 4-méthoxy-4'-n-butyl-dibenzoyl-méthane | 358 | 34720 | A |
| | | | |
| solvant: éthanol | | | |

Les préparations cosmétiques contiennent presque toujours un mélange de filtres UV-A et UV-B,afin de réaliser la plus large protection possible.

Les qualités idéales des filtres solairesUV-A et UV-B sont les suivantes:
1) une absorbabilité de la lumière UV-A et UV-B supérieure à ε max.20000,
2) une bonne photostabilité,
3) une bonne stabilité chimique,
4) une bonne fixation au stratum corneum,
5) une bonne incorporation à la composition cosmétique,
6) une bonne inocuité,

La présente invention concerne les substances organiques objet des revendications 1 à 6.Cessubstances sont utilisables en tant que filtres solaire,d'écrans solaires,de protecteurs des denrées alimentaires et des matériaux contre les altérations provoquées par la lumière ultra-violette. La structure des substances organiques a été choisie en fonction
1) de leur grand pouvoir absorbant la lumière UV-A et UV-B (ε max suprérieur à 20000),
2) de leur photostabilité,
3) de leur parentée à des substances déjà utilisées comme filtres solaires,dont on a reconnu à l'échelle mondiale,leur inocuité sanitaire,
4) leur résistivité en milieu aqueux,
5)leur appartenance à une même famille de composés dont de faible variation permet de moduler leurs propriétés UV-A et UV-B.

Une bonne absorbabilité des lumières UV-A et UV-B requiert de la part des substances organiques un degré d'insaturation qui augmente en passant des structures UV-B aux structures UV-A.

La modulation des propriété absorbantes,exprimées en ε max, requiert aussi la présence de fonctions organiques à effet inductif (donneur d'électrons)sur le système insaturé,par ex.les fonctions alkoxy,hydroxy,amino,alkylamino, et en position favorable par rapport à ces fonctions des groupements d'atomes(attireurs d'électrons),par ex. les fonctions carboxy,carboalkoxy,carbamides,cétonique,sulfonique,nitrile.

Les structures insaturée au carbone,non cyclique, par ex. les dérivés de l'éthylène,subissent l'isomérie cis-trans,dont souvent l'équilibre est déplacé par la lumière ultra-violette vers l'isomère géomètrique au pouvoir absorbant le plus bas.Une insaturation entre deux restes aromatiques rèalisée par deux carbones n'est donc pas souhaitable.Par contre,une fonction comprenant deux centres polarisables entre deux restes aromatiques est susceptible de créer une conjugaison dont l'état excité par les photons ultraviolets pourrait être d'énergie relativement peu élevée par rapport à l'état fondamental.Cet effet provoquera selon les fonctions présentes un déplacement hypsochromique ou bathochromique voire auxochromique des valeurs spectrales c-à-d il permettra le choix d'une structure appropriée pour la préparation d'un filtre solaire souhaité.

Nous avons choisi pour la fonction intermédiaire,polarisable,entre une reste cinnamyle et un reste phényle,la fonction-CONH-,réalisant les N-phényl-cinnamamides.Leur pouvoir d'absorption de la lumière entre 280 et 350 nm(UV-B et UV-A) est trés élevé.La position de leur λ max.peut être choisie choisie en vertu des fonctions donneur et accepteur d'électrons placées séparément sur le reste cinnamyl d'un côté et sur le reste phényl de l'autre.Pour la raison de la meilleure transmission de cet effet à travers toute la molécule,nous préférons les positions para et ortho par rapport à la place de la fonction intermédiaire-CONH-
Les deux structures générales des N-phényl-cinnamamides selon un mode particulier de l'invention sont représentées dans le taleau II.

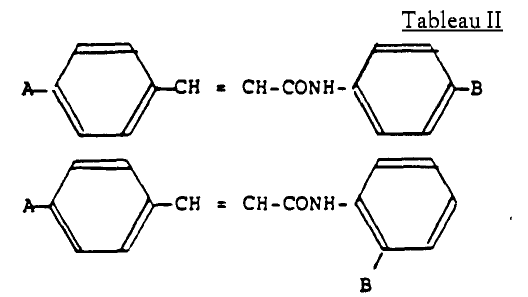

Lorsque à partir de la fonction donneur d'électrons A,la fonction B accepteur d'électrons permet d'étendre la polarisation au reste phényle,l'effet bathochromique augmente(déplacement vers ou dans les UV-A);lorsque la fonction A est moins donneur d'électrons l'effet hypsochromique se manifeste( déplacement vers ou dans les UV-B).
Ainsi,dans la même famille de subtances,les N-phényl-cinnamamides, on peut confectionner un filtre solaire à trés fort pouvoir absorbant de la lumière ultra-violette choisie.Ces propriétés confèrent aux N-phényl-cinnamamides,comme fitres solaires,protecteurs des denrées alimentaires et des matériaux contre les altérations provoquées par la lumière ultra-violette,une qualité de protection que l'on ne connaît pas chez les filtres solaires commercialisés(voir le tableau I).

Des N-phénylcinnamamides ont été déjà proposées comme filtres solaires:la N-(4-sulfophényl)-cinnamamide(et ses sels) est décrite dans EP-A 950097.Elle possède une absorption UV-B (λ max.= 300 nm), et son intensité d'absorption est relativement faible (ε = 25100). La N-(4-carbéthoxy-phényl)-4-diméthyl-amino-cinnamamide décrite dans JP A-74 69,847 absorbe dans les UV A, le proche visible. Sa coloration le rend impropre à l'usage cosmétique.

### Les exemples:

La synthèse des N-phényl-cinnamamides a été effectueé à partir des dérivé appropriés de l'acide cinnamique dont la fonction carboxy a été transformée en chlorure d'acide ou anhydride d'acide du anhydride mixte ou en esters activés ou azide ou en un dérivé d'une carbodiimide réagissant avec la fonction amino phényl substitué ou non,dans un solvant anhydre.Les produits sont cristallisés dans l'eau et recristallisés dans le solvant indiqué dans la description des N-phényl-cinnamamides.
Les analyses ont portés sur la composition C,H,N,S;les points de fusion sont déterminés avec l'appareil de Kofler(Reichert);les spectres UV ont été établis avec le spectromètre Cary 2300;les spectres IR ont été obtenus avec le spectromètre 735 B de Perkin-Elmer.

### Exemple 1 N-(4-carbéthoxy-phényl)-4-méthoxy-cynnamamide

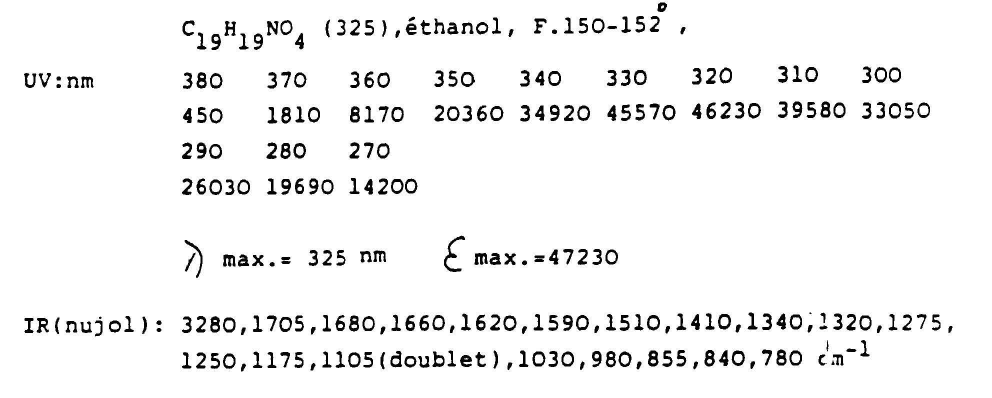

### Exemple 2 N-(2-carbéthoxy-phényl)-4-méthoxy-cinnamamide

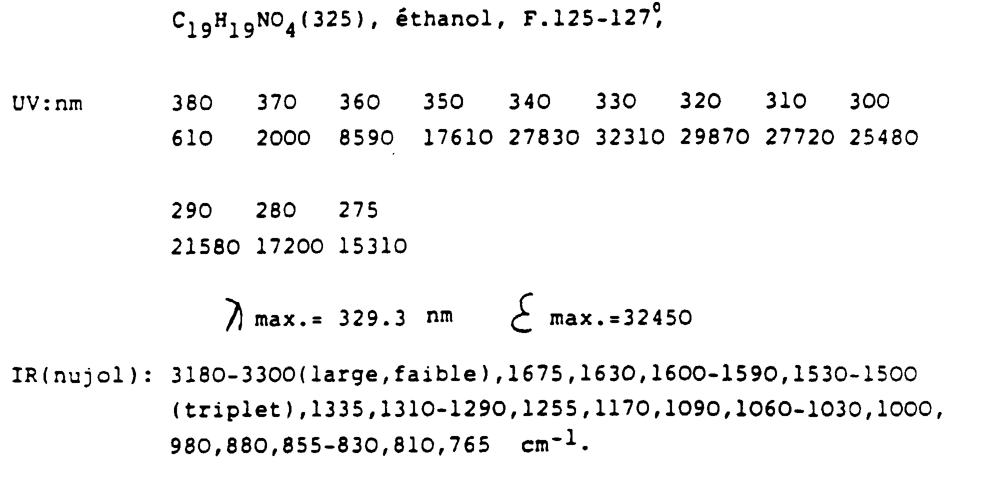

### Exemple 3 N-(4-carboxy-phényl)-4-méthoxy-cinnamamide

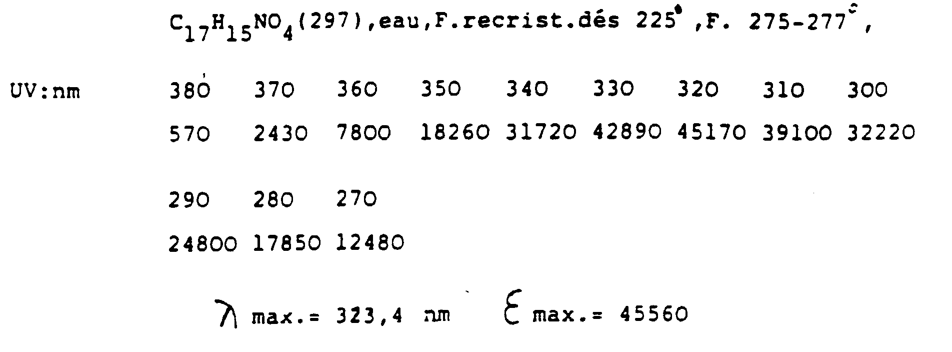

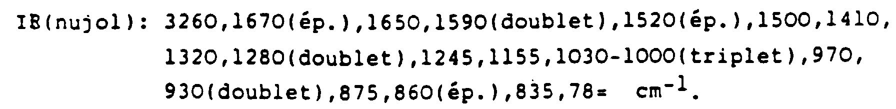

### Exemple 4 N-(4-sulfo-phényl)-4-méthoxy-cinnamamide(sel de triéthylamine)

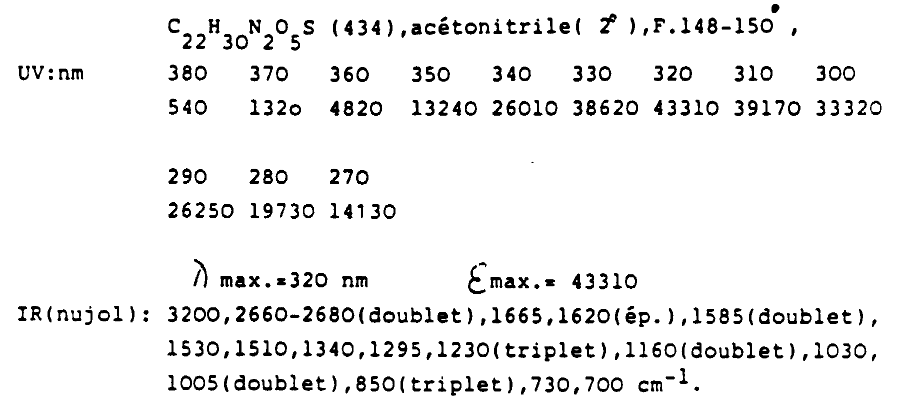

### Exemple 5 N-(4-benzoyl-carboxamido-phényl)-4-méthoxy-cinnamamide

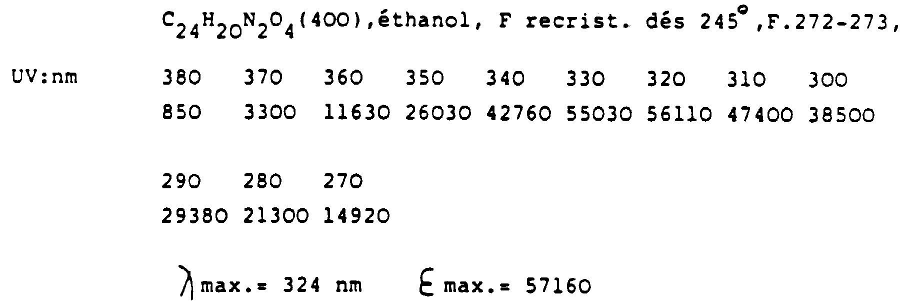

## Revendications

1. Les N-phényl-cinnamamides de la formule I,utilisés en qualité de filtres solaires et d'écrans solaires en cosmétique et applications topiques médicales,utilisés en qualité d'agents de protection des denrées alimentaires et des matériaux contre les dommages provoqués par la lumière ultra-violette, 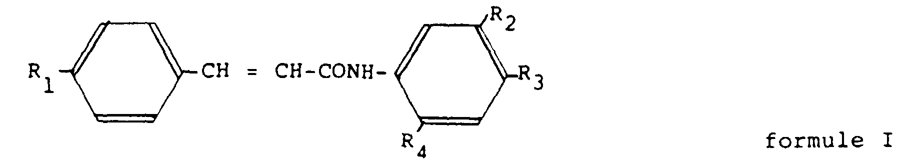 dans laquelle R₁ est un hydrogène,une fonction alkoxy,une fonction phénoxy,une fonction dialkyl-amine,
dans laquelle R₂est un hydrogène,une fonction hydroxy,une fonction acide carboxylique,une fonction ester carboxylique,une fonction sulfonique,
dans laquelle R₃ est une fonction carboxylique modifiée:
une fonction acide carboxylique à l'exception du cas dans lequel R₁ est une fonction dialkylamino,
une fonction ester carboxylique à l'exception du cas dans lequel R₁ est une fonction dialkylamino,
une fonction carboxamide,
une fonction carboxamide avec un acide aminé,
une fonction amide peptidique,
une fonction amide avec une amine aromatique
une fonction amide N-acylée,
une fonction cétonique aliphatique,
une fonction cétonique aromatique,
une fonction -dicétonique,
une fonction sulfonique à l'exception du cas dans lequel R₁ est un hydrogène,
une fonction sulfamyle,une fonction sulfoxyde,une fonction sulfone,
une fonction nitrile,
un reste 2-benzimidazole substitué ou non
un reste 2-benzothiazole substitué ou non.
dans laquelle R₄ est un hydrogène,une fonction hydroxy,une fonction alkoxy jusqu'à 6 atomes de carbone,une fonction acide carboxylique,une fonction ester carboxylique si R₁ n'est pas un alkoxy de 1 à 4 atomes de carbone.

2. Une N-phényl-cinnamamide de la formule II,selon la revendication 1,caractérisée par ses propriétés de filtre solaire UV-B,de protection des denrées alimentaires et des matériaux contre les dommages provoqués par la lumière ultra-violette,

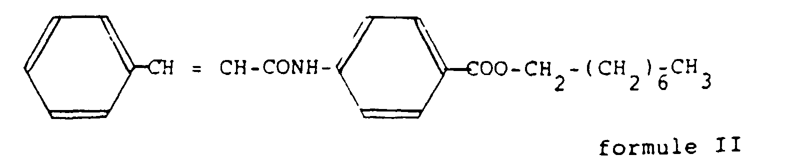

3. Une N-phényl-cinnamamide de la formule III,selon la revendication 1,caractérisée par ses propriétés de filtre solaire UV-A et UV-B,de protection des denrées alimentaires et des matériaux contre les dommages provoqués par la lumière ultra-violette,

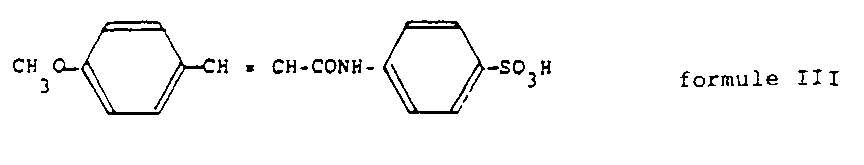

4. Une N-phényl-cinnamamide de la formule V,selon la revendication1,caratérisée par ses propriétés de filtre solaire UV-A et UV-B,de protection des denrées alimentaires et des matériaux contre les dommages provoqués par la lumière ultra-violette,

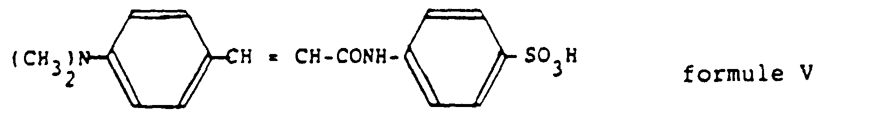

5. Une N-phényl-cinnamamide de la formule VI,selon la revendication 1,caractérisée par ses propriétés de filtre solaire UV-A et UV-B,de protection des denrées alimentaires et des matériaux contre les dommages provoqués par la lumière ultra-violette,

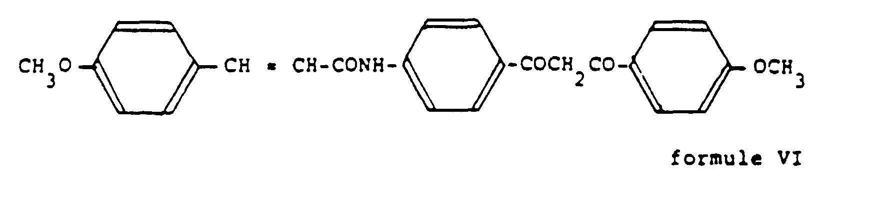

6. Une N-phényl-cinnamamide de la formule VII,selon la revendication 1,caractérisée par ses propriétés de filtre solaire UV-A et UV-B,de protection des denrées alimentaires et des matériaux contre les dommages provoqués par la lumière ultra-violette,

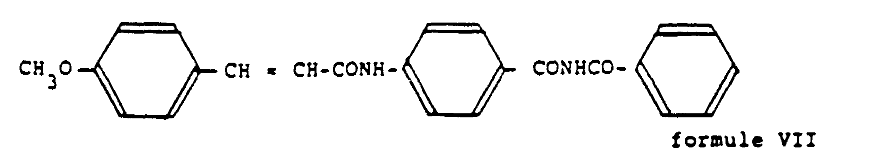

7. Usage de la N-(4-carboxy-phényl)-4-méthoxy-cinnamamide, de la N-(4-carbéthoxy-phényl)-4-méthoxy-cinnamamide et de la N-(4-carbo-2-éthyl-hexyloxy-phényl)-4-méthoxy-cinnamamide en qualité de filtres solaire UV-AB,pour des applications dans des formulations cosmétiques.

8. Usage de la la N-(4-(6-méthyl-benzothiazolyl-2-phényl)-4-méthoxy-cinnamamide en qualité de filtres solaires UV-A, pour des applications dans des formulations cosmétiques.

9. Usage de la N-(4-carbéthoxy-phényl)-cinnamamide en qualité de filtres solaires UV-B,pour des applications dans des formulations cosmétiques

10. Usage des N-phényl-cinnamamides suivant les revendications 1 à 6 en qualité d'agents de protection des matériaux contre les dommages provoqués par la lumière ultra-violette.

## Patentansprüche

1. Die *N-phenyl-cinnamide* der Formel I, als Sonnenfilter und als Sonnenschutz in der Kosmetik und für äusserliche medikale Anwendungen gebraucht, als Schutzmittel für Lebensmittel und Materialien gegen durch ultraviolettes Licht hevorgerufene Schäden gebraucht, 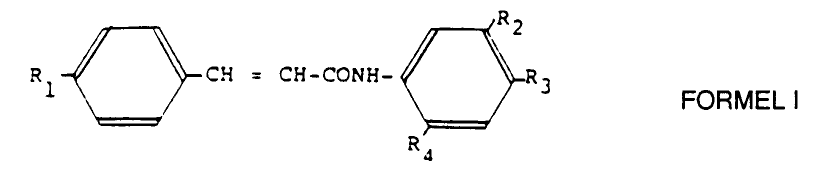
wo R1 ein *Wasserstoff, eine Alkoxy-, Phenoxy-, Dialkylaminfunktion* ist,
wo, R2 ein *Wasserstoff*, *eine Hydroxy-, eine Carbonsäure-, eine Carbonsäureester,eine Sulfonsäurefunktion* ist,
wo R3 eine veränderte *Carboxylfunktion* ist:
*eine Carbonsäurefunktion* ausser in dem Fall, wenn R1 eine *Dialkylaminfunktion* ist,
eine *Carbonsäureesterfunktion* ausser in dem Fall, wenn R1 eine *Dialkylaminfunktion* ist,
eine *Carboxamidfunktion,*
eine *Carboxamidfunktion mit einer Aminosäure,*
*eine peptidische Amidfunktion,*
*eine Amidfunktion mit einem aromatischen Amin,*
*eine n-acylierte Amidfunktion,*
*eine aliphatische Ketonfunktion,*
*eine aromatische Ketonfunktion,*
*eine Diketonfunktion,*
*eine Sulfonsäurefunktion* ausser in dem Fall, wenn R1 ein Wasserstoff ist,
*eine Sulfamylfunktion, eine Sulfoxydfunktion, eine Sulfonfunktion,*
*eine Nitrilfunktion,*
*ein substituierter oder nicht substituierter 2-Benzimidazolrest,*
*ein substituierter oder nicht substituierter 2-Benzothiazolrest.*
wo R4 ein *Wasserstoff, eine Hydroxylfunktion, eine Alkoxyfunktion mit bis zu 6 Kohlenstoffatomen,* eine *Carbonsäurefunktion, eine Carbonsäureesterfunktion* in dem Fall, wenn R1 kein *Alkoxy* mit 1 bis 4 Kohlenstoffatomen ist.

2. Ein *N-Phenylcinnamid* der Formel II, gemäss dem Patentanspruch 1, durch seine Eigenschaften als UV-B Sonnenfilter gekennzeichnet, als Schutz von Lebensmittel und Materialien gegen durch ultraviolettes Licht hevorgerufene Schäden,

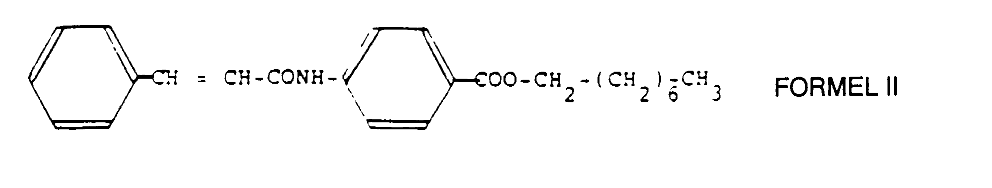

3. Ein *N-Phenylcinnamid* der Formel III, gemäss dem Patentanspruch 1, durch seine Eigenschaften als UV-A und UV-B Sonnenfilter gekennzeichnet, als Schutz von Lebensmittel und Materialien gegen durch ultraviolettes Licht hevorgerufene Schäden,

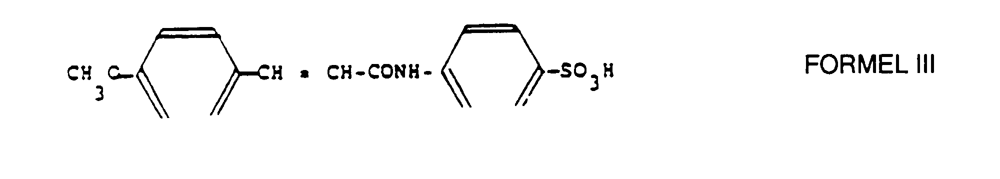

4. Ein *N-Phenylcinnamid* der Formel V, gemäss dem Patentanspruch 1, durch seine Eigenschaften als UV-A und UV-B Sonnenfilter gekennzeichnet, als Schutz von Lebensmittel und Materialien gegen durch ultraviolettes Licht hevorgerufene Schäden,

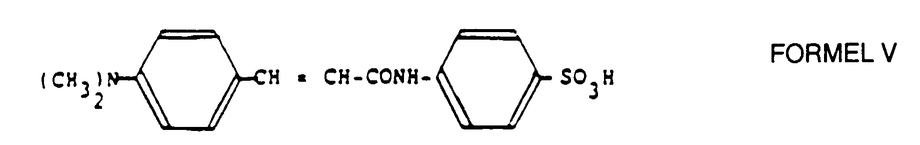

5. Ein *N-Phenylcinnamid* der Formel VI, gemäss dem Patentanspruch 1, durch seine Eigenschaften als UV-A und UV-B Sonnenfilter gekennzeichnet, als Schutz von Lebensmittel und Materialien gegen durch ultraviolettes Licht hevorgerufene Schäden,

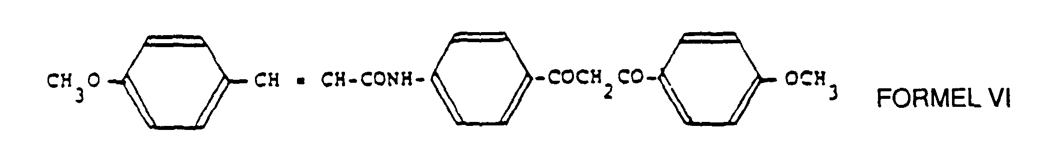

6. Ein *N-Phenylcinnamid* der Formel VII, gemäss dem Patentanspruch 1, durch seine Eigenschaften als UV-A und UV-B Sonnenfilter gekennzeichnet, als Schutz von Lebensmittel und Materialien gegen durch ultraviolettes Licht hevorgerufene Schäden,

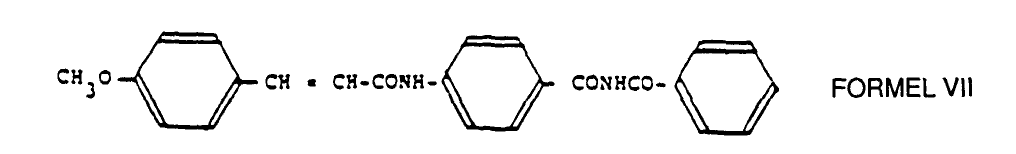

7. Verwendung des *N-(4-carboxy-phenyl) 4-methoxycinnamid,* des N-(4carbethoxyphenyl) *4-methoxycinnamid, und des N-(4-carbo-2 ethyl-hexyloxy-phenyl) 4-methoxycinnamid* als UV-AB Sonnenfilter für Anwendungen in kosmetischen Formulierungen.

8. Verwendung des *N-(4-(6-methyl-benzothiazolyl-2-phenyl) 4-methoxycinnamid* als UV-A Sonnenfilter für Anwendungen in kosmetischen Formulierungen.

9. Verwendung des *N-(4-carbethoxy-phenyl) 4-methoxycinnamid* als UV-B Sonnenfilter für Anwendungen in kosmetischen Formulierungen.

10. Verwendung der *N-phenyl-cinnamide* gemäss den Patentansprüchen 1 bis 6 als Schutzmittel von Materialien gegen durch ultraviolettes Licht hervorgerufene Schäden.

## Claims

1. The N-phenyl-cinnamides having the formula I used as a cosmetic solar filters and sunscreens and as agents protecting food products and materials from damage caused by ultraviolet-light, 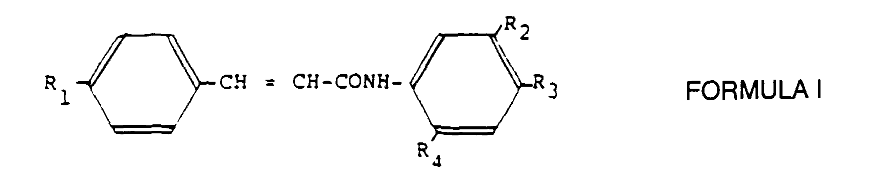
wherein R1 is selected from a hydrogen, the group alkoxy, the group phenoxy, group dialkylamino,
wherein R2 is selected from a hydrogen, a alkoxy function, carboxylic acid function, a carboxylic ester function, a sulfonic acid function,
wherein R3 is selected from the group consisting of a carboxylic acid function except the case in which R1 is a dialkylamino function, a carboxylic ester function except the case wherein R1 is a dialkylamino function, a carboxamide function, a carboxamide function with an amino acid, a peptidic amide function, an amide function with an aromatic amine, a N-acylated amide function, an aliphatic ketonic function, an aromatic ketonic function, a diketone function, a sulfo function except when R1 is a hydrogen, a sulfoxyde function, a sulfone function, a nitrile function, a substituted or unsubstituted 2-benzimidazole residue, a substituted or unsubstituted 2-benziothiazole residue,
wherein R4 is selected from the group consisting of a hydrogen, a hydroxy function, a alkoxy function of up to 6 carbons, a carboxylic ester function if R1 is not a C 1-4 alkoxy function,

2. The N-phenyl-cinnamamide having the formula II, follower claim 1, characterized by his sunscreen UV-B properties as agent protecting food products and materials from damage caused by ultraviolet-light

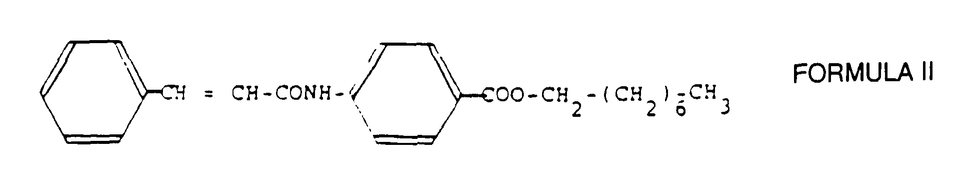

3. The N-phenyl-cinnamide having the formula III, follower the claim 1 characterised by his sunscreen UV-A and UV-B properties, as agent protecting food products and materials from damage caused by ultra-violet light

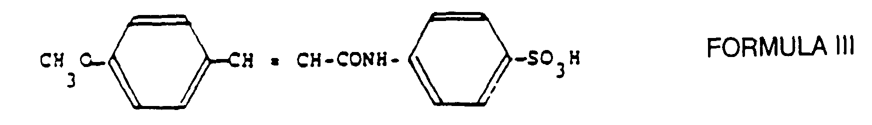

4. The N-phenyl-cinnamamide having the formula V, follower the claim 1, characterised by his sunscreen UV-A and UV-B properties, as agent protecting food products and materials from damage caused by ultra-violet lights.

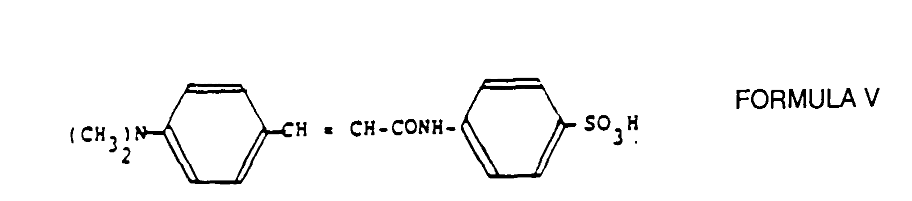

5. The N-phenyl-cinnamamide having the formula VI, follower the claim 1, characterised by his sunscreen UV-A and UV-B properties, as agent protecting food products and materials from damage caused by ultra-violet light,

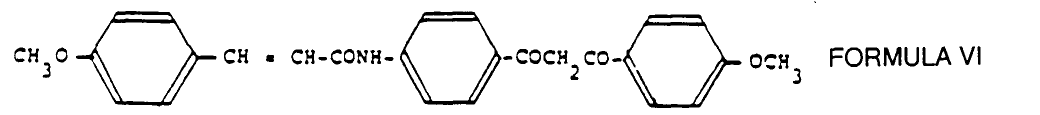

6. The N-phenyl-cinnamamide having the formula VII, follower the claim 1, characterised by his sunscreen UV-A and UV-B properties, as agent protecting food products and materials from damage caused by ultra-violet light

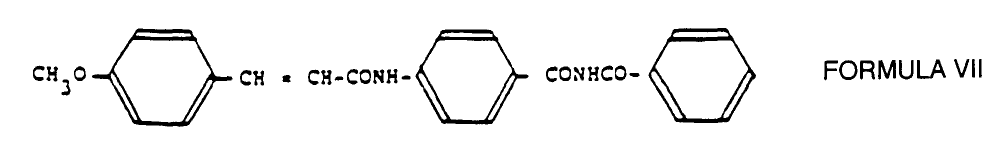

7. Use of the N-(4-carboxy-phenyl)-4-methoxy-cinnamamide and of the N(-4-carbo-2-ethyl-hexyloxy-phenyl)-4-methoxy-cinnamamide as sunscreens UV-AB, for use in cosmetic formulations

8. Use of the N-(4-(6 methyl-benzothiazlyl-2-phenyl)-4-methoxy-cinnamamide as sunscreen UV-A for use in cosmetic formulations.

9. Use of the N-(4-carbethoxy-phenyl)-cinnamaide as sunscreen UV-B, for use in cosmetic formulations

10. Use of the N-phenyl-cinnamamides follower claims 1 to 6, as agents protecting materials from damage caused by ultra-violet light.
